# EUROPEAN PATENT SPECIFICATION

(11) **EP 2 805 742 B1**
(45) Date of publication and mention of the grant of the patent: **26.04.2017**
(21) Application number: 13741257.3
(22) Date of filing: 23.01.2013
(51) Int. Cl.: A61M 25/00, A61M 25/14, A61M 25/10, A61M 25/01

(54) **BALLOON CATHETER**
BALLONKATHETER
CATHÉTER À BALLONNET

(30) Priority: 26.01.2012 JP 2012013996
(43) Date of publication of application: 26.11.2014
(73) Proprietor: Japan Lifeline Co., Ltd., Tokyo 140-0002 (JP)
(72) Inventor: OKAWA, Yasuhiro, Tokyo 140-0002 (JP)
(74) Representative: Prüfer & Partner mbB Patentanwälte · Rechtsanwälte
(86) International application number: PCT/JP2013/051296
(87) International publication number: WO 2013/111774

(56) References cited:
- WO-A1-2006/126642
- WO-A1-2011/036667
- JP-A- 2010 527 258
- JP-A- 2011 515 150
- US-A1- 2007 088 257
- US-A1- 2008 171 979
- US-A1- 2008 177 227
- US-B1- 7 195 611

## Description

### [Technical Field]

The present invention relates to a balloon catheter.

### [Background Art]

In the current medical treatment, catheter operations using catheters have been widely carried out. These catheter operations include those using balloon catheters. The balloon catheter has a balloon-shaped part at a tip thereof and is for carrying out treatment of an occluded part or stenosed part of a blood vessel by expanding the balloon-shaped part in the vessel. The balloon catheters include one that is inserted in a blood vessel to expand an occluded part or a stenosed part in the blood vessel. The balloon catheter of this type includes one in which a guide wire port is in a tip side as shown in Patent Literature 1.

In this Patent Literature 1, a balloon catheter having a guide wire port is prepared by joining two outer tubes (a first shaft and a second shaft) and an inner tube (guide wire shaft) by heat in order to create the guide wire port.

US 2007/088257 A1, WO 2011/036667 A1, US 2008/171979 A1 and US 2008/177227 A1 disclose a balloon catheter including an expandable balloon, a hypotube, a tube member and an inner tube according to claim 1.

US 7,195,611 B1 discloses a balloon catheter corresponding to the preamble of claim 1.

### [Citation List]

### [Patent Literature]

[Patent Literature 1] Japanese Patent Application Laid-Open No. 2008-259695

### [Summary of Invention]

### [Technical Problem]

Incidentally, if the two outer tubes and the inner tube are joined by heat in the above described manner, below problems occur. That is, in the vicinity of the guide wire port, the two outer tubes and the inner tube are joined by thermal fusion to form one tube; however, if the thermal fusion is carried out, there is a problem that the fused part is hardened. If such a hardened part is generated, when a tubular catheter main-body part is inserted in the blood vessel, the manipulability in manipulation of the catheter main-body part is worsened.

The present invention has been accomplished in view of the above described circumstances, and an object thereof is to provide a balloon catheter having good manipulability.

### [Solution to Problems]

In order to solve the above described problems, a balloon catheter in accordance with the present invention is provided as defined in claim 1.
Further advantages are achieved by the embodiments indicated by the dependent claims.

### [Advantageous Effect of Invention]

According to the present invention, a balloon catheter having good manipulability can be provided.

### [Brief Description of Drawings]

[FIG 1] FIG. 1 is a drawing showing an overall structure of a balloon catheter according to an embodiment of the present invention.
[FIG. 2] FIG. 2 is a partial cross-sectional view showing a structure of a shaft main-body part in the balloon catheter of FIG. 1.
[FIG. 3] FIG. 3 is a partial cross-sectional view showing details of a structure of a distal tube part in the balloon catheter of FIG. 1.
[FIG. 4] FIG. 4 is a cross-sectional view showing a state in which the distal tube part is cut along a line A-A of FIG. 3.
[FIG. 5] FIG. 5 is a cross-sectional view in which peel-off occurs to form burrs between an outer layer and an inner layer in a proximal end side of an inner tube in a conventional structure.

### [Description of Embodiment]

Hereinafter, a balloon catheter 10 according to an embodiment of the present invention will be explained based on drawings.

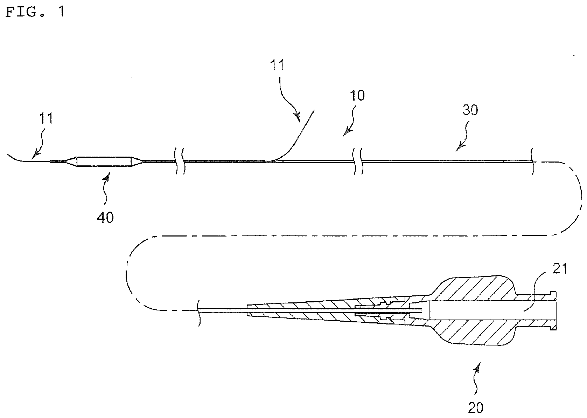

FIG. 1 is a drawing showing an overall structure of the balloon catheter 10. The balloon catheter 10 in the present embodiment is that of a type in which it is inserted in a blood vessel along a guide wire 11. Note that, in the below description, the general idea of the balloon catheter 10 is described as the balloon catheter not including the guide wire 11 described later. However, the general idea of the balloon catheter 10 may include the guide wire 11.

As shown in FIG. 1, the balloon catheter 10 has a hub 20, a shaft main-body part 30, and a balloon 40.

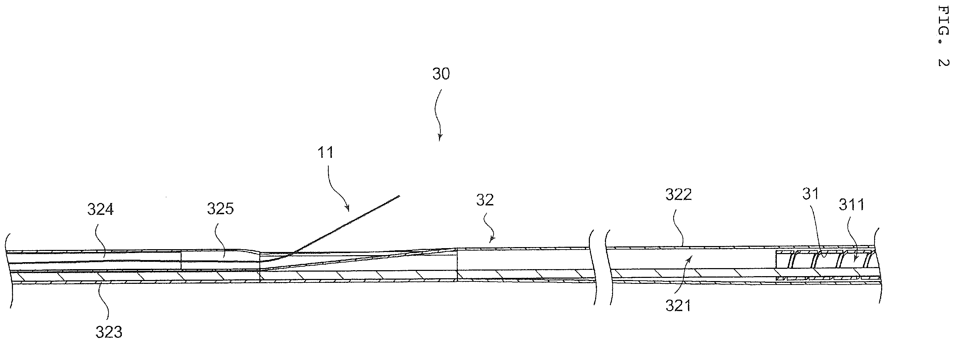

The hub 20 is a part which is held in an operation, the hub 20 is provided with a communication pipe line 21 which is a hollow part, and the communication pipe line 21 is communicated with tube pipe lines 311 and 321 of the shaft main-body part 30 (see FIG. 2).

As shown in FIG. 2, the shaft main-body part 30 is provided with a hypotube part 31, a distal tube part 32, and a core wire 33. The hypotube part 31 is formed of a material such as metal having a higher rigidity than that of the distal tube part 32, and the proximal end side thereof is connected to the hub 20. The hypotube part 31 is provided with the hollow tube pipe line 311 along the longitudinal direction of the hypotube part 31. The tube pipe line 311 is communicated with the communication pipe line 21 of the hub 20.

The distal tube part 32 is formed of a material having flexibility, the proximal end side thereof is connected to the hypotube part 31, and the distal end side thereof is connected to the balloon 40. The distal tube part 32 is also provided with the hollow tube pipe line 321 along the longitudinal direction thereof. The tube pipe line 321 is communicated with the tube pipe line 311 of the hypotube part 31. Note that details of the structure of the distal tube part 32 will be described later.

The core wire 33 communicates the tube pipe line 311 of the above described hypotube part 31 and the tube pipe line 321 of the distal tube part 32. The core wire 33 is provided in the tube pipe lines 311 and 321 in order to, for example, prevent a kink (bend) of the shaft main-body part 30 and improve workability in push-in of the balloon 40 and the shaft main-body part 30 into a blood vessel along the guide wire 11. Note that, the material of the core wire 33 is only required to be metal, but is preferred to be a stainless alloy, a cobalt-chrome alloy, or a nickel-titanium alloy because of processability or safety with respect to a living body.

The interior of the balloon 40 is communicated with the tube pipe lines 311 and 321, and the balloon 40 is a member which can be inflated like a balloon by supplying liquid to the interior via the tube pipe lines 311 and 321. The balloon 40 can expand a stenosed part of a blood vessel, for example, by inflating cylindrically.

### <About Details of Structure of Distal Tube Part 32>

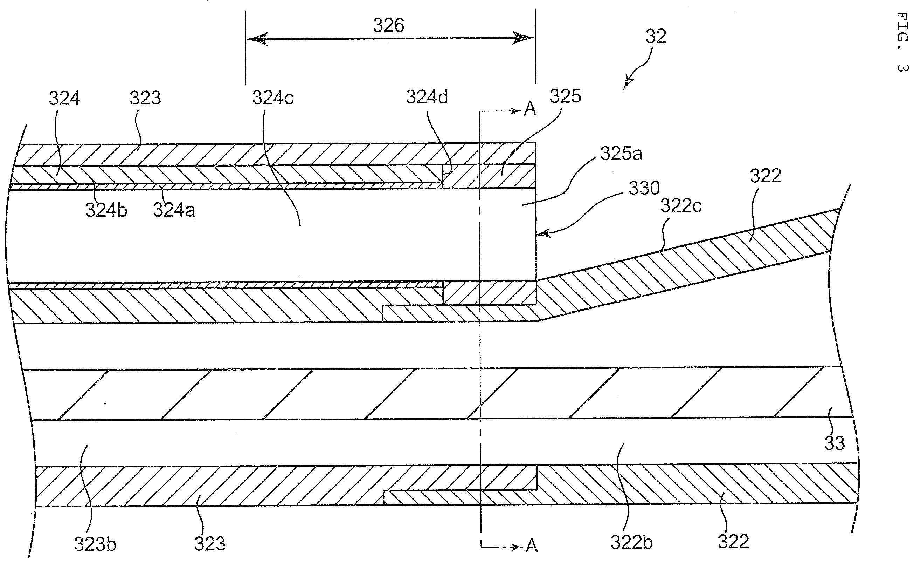

Subsequently, details of the structure of the distal tube part 32 will be described below. As shown in FIG. 3, the distal tube part 32 in the present embodiment has a first outer tube 322, a second outer tube 323, a guide wire port 330, an inner tube 324, and a connection tube 325.

The proximal end side of the first outer tube 322 is connected to the above described hypotube part 31. More specifically, the proximal end side of the first outer tube 322 is in a state that the outer peripheral side of the hypotube part 31 is positioned in the inner peripheral side of the first outer tube 322, and the first outer tube 322 and the hypotube part 31 are joined with each other. The material of the first outer tube 322 is formed by using a polyamide-based resin as a material.

Note that, the polyamide-based resin is typified by, for example, block copolymers using various fatty series or aromatic polyamides such as nylon 6, nylon 64, nylon 66, nylon 610, nylon 612, nylon 46, nylon 9, nylon 11, nylon 12, N-alkoxymethyl modified nylon, hexamethylenediamine-isophthalate condensation polymers, and metaxyloyldiamine-adipic acid condensation polymers as a hard segment and using polymers such as polyester and polyether as a soft segment; in addition to that, the polyamide-based resin is a general idea that includes polymer alloys of the above described polyamides and a flexible resin (polymer blend, graft polymerization, random polymerization, etc.), the above described polyamides softened, for example, by a plasticizer, and mixtures thereof. However, aliphatic polyamides (nylon-based resins) are preferred among aliphatic polyamides and aromatic polyamides.

A material which is suitably used as the first outer tube 322 is, for example, "PEBAX" (registered trademark) produced by ARKEMA from the viewpoint of processability thereof. Note that, the Shore D hardness of the first outer tube 322 is, for example, 72, which is provided to be harder than the second outer tube 323.

Moreover, the second outer tube 323 is provided in the distal end side (the balloon 40 side) of the above described first outer tube 322, and a lumen part 322b of the first outer tube 322 and a lumen part 323b of the second outer tube 323 are communicated with each other.

The second outer tube 323 is formed by using the above described polyamide-based resin as a material as well as the above described first outer tube 322. The second outer tube 323 may have a structure in which only one layer of a tube member composed of the polyamide-based resin is present, but may have a structure in which two or more layers of a tube member composed of the polyamide-based resin are present (illustration omitted). Note that, also in the first outer tube 322, two or more layers of a tube member composed of the polyamide-based resin may be present.

If the second outer tube 323 has a two-layer structure composed of the polyamide-based resin, the Shore D hardness of a tube body in an inner layer side is, for example, 63, and the Shore D hardness of a tube body in an outer layer side is, for example, 72. Since the tube body having the lower hardness than that of the first outer tube 322 is present in the inner layer side of the second outer tube 323 in this manner, the hardness of the second outer tube 323 is smaller than that of the first outer tube 322.

Note that, the first outer tube 322 and the second outer tube 323 correspond to examples of a tube member referred to in claims.

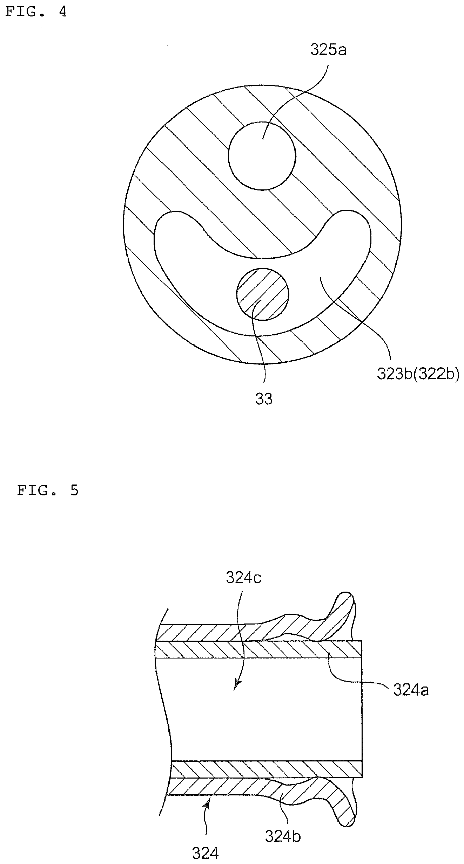

In the lumen part 323b of the second outer tube 323, the core wire 33 and the inner tube 324 are disposed. In the second outer tube 323, at least part of the inner tube 324 is disposed between the balloon 40 and the guide wire port 330. The inner tube 324 has a two-layer structure (see FIG. 5) for improving slipping with respect to the guide wire 11, and the frictional coefficient of an inner layer 324a thereof is lower than the frictional coefficients of the first outer tube 322 and the second outer tube 323. More specifically, the inner layer 324a of the inner tube 324 uses a polyethylene resin or a fluorine-based resin as a material, an outer layer 324b of the inner tube 324 uses a polyamide-based resin as a material, and the inner tube 324 is formed by joining these via an adhesive agent.

Herein, the outer layer 324b of the inner tube 324 composed of the polyamide-based resin is also suitable to be, for example, "PEBAX" (registered trademark) produced by ARKEMA from the viewpoint of processability thereof. The Shore D hardness of the outer layer 324b of the inner tube 324 is, for example, 63, but may be hardness other than that.

In the inner tube 324, a fused part 326, which is fused with the second outer tube 323 is present. In the fused part 326, the outer layer 324b of the inner tube 324 and the second outer tube 323 are melted and mixed with each other and are in an integrated state. By virtue of this, the joint therebetween is firm. However, at the inner layer 324a of the inner tube 324 using the polyethylene resin or the fluorine-based resin as a material, a state that, even when the outer layer 324b is melted, the inner layer 324a is not melted occurs due to a difference of the melting point with respect to the polyamide-based resin. Therefore, together with the second outer tube 323, the outer layer 324b serves as a main constituent element of fusion.

The connection tube 325 is joined with the proximal end side of the inner tube 324, and the connection tube 325 is disposed in the vicinity of the guide wire port 330. More specifically, the outer layer 324b of the inner tube 324, the connection tube 325, the first outer tube 322, and the second outer tube 323 are joined so that the lumen part 324c of the inner tube 324 is communicated with a lumen part 325a of the connection tube 325. More specifically, the proximal end side of the inner tube 324 with the distal end side of the connection tube 325 and the distal end side of the first outer tube 322 with the proximal end side of the second outer tube 323 are melted and mixed to provide the fused part 326 in an integrated state. The fused part 326 has a cross section as shown in FIG. 4.

The connection tube 325 is formed by using a polyamide-based resin as a material as well as the above described first outer tube 322. However, the connection tube 325 is formed of a material having lower hardness than that of the first outer tube 322, the second outer tube 323, and the inner tube 324, and the Shore D hardness of the connection tube 325 is, for example, 55. Note that the Shore D hardness of the connection tube 325 is not limited to 55, but may be any value as long as it is lower than the Shore D hardness of the first outer tube 322, the second outer tube 323, and the inner tube 324.

The Shore D hardness of the connection tube 325 is preferred to be lower than the Shore D hardness of the outer layer 324b of the inner tube 324, but may be higher than the Shore D hardness of the outer layer 324b of the inner tube 324. However, the Shore D hardness of the connection tube 325 is preferred to be smaller than that of both of the first outer tube 322 and the second outer tube 323.

As the preferred Shore D hardness, the Shore D hardness of the first outer tube 322 is within a range of 80 to 63, the Shore D hardness of the second outer tube 323 is within the range of 80 to 63, and the Shore D hardness of the outer layer 324b of the inner tube 324 is within the range of 72 to 50. If the Shore D hardness thereof is within them, the Shore D hardness of the connection tube 325 is preferred to be within the range of 63 to 25. If the Shore D hardness of the connection tube 325 is within the range of 62 to 25, the hardness difference of the Shore D hardness thereof with respect to the first outer tube 322 and the second outer tube 323 is 1 to 38. If such a hardness difference is obtained, the connection tube 325 well follows bending, etc. of the guide wire 11, and the manipulability of the balloon catheter 10 can be further improved.

A part in the distal end side of the connection tube 325 is joined with the inner tube 324. By this joint, the inner layer 324a composed of the polyethylene resin or the fluorine-based resin of the inner tube 324 is prevented from being exposed to outside.

The guide wire port 330 in the proximal end side of the connection tube 325 enables the guide wire 11 to be inserted/removed to/from the lumen part 325a of the connection tube 325 and the lumen part 324c of the inner tube 324 and is provided on a side surface of the second outer tube 323. However, the general idea of the guide wire port 330 may include a guide part 322c, which is formed by a slope in the upper side of the first outer tube 322 in FIG. 3.

### <About Effects>

According to the balloon catheter 10 having the above described structure, the guide wire port 330 is provided with the connection tube 325 having hardness lower than at least that of the first outer tube 322 and the second outer tube 323. Therefore, the part hardened by thermal fusion can be caused to be flexible, and the manipulability of the balloon catheter 10 can be improved.

More specifically, conventionally, in the guide wire port 330, two outer tubes (the first outer tube 322 and the second outer tube 323) and the inner tube 324 have been heated and thermally fused. In the thermal fusion, the inner layer 324a of the inner tube 324 uses a polyethylene resin or a fluorine-based resin as a material, and the inner layer 324a is harder than the outer layer 324b of the inner tube 324; therefore, the inner layer 324a is melted and flowed by heating, and, when hardened by cooling thereafter, the part hardened by thermal fusion is hardened. As a result, shaft balance becomes bad, and the manipulability of the balloon catheter 10 is worsened.

However, in the present embodiment, the connection tube 325 is disposed in the proximal end side of the inner tube 324 and at the guide wire port 330, and the connection tube 325 serves as an outlet/inlet of the guide wire 11. The hardness of the connection tube 325 is lower than the hardness of at least the first outer tube 322 and the second outer tube 323. By virtue of this, the connection tube 325 well follows bending, etc. of the guide wire 11; therefore, problems caused in manipulations due to the hardness of the outlet/inlet of the guide wire 11 with respect to the manipulations of the guide wire 11 can be prevented. As described above, in the invention of the present embodiment, the manipulability of the guide wire 11 can be improved.

In the conventional structure (more specifically, a structure in which the inner tube 324 extends to the position of the connection tube 325 in FIG. 3, and the connection tube 325 is not present), in the inner tube 324, melting points are different between that of the inner layer 324a using the polyethylene resin or the fluorine-based resin as a material and that of the outer layer 324b using the polyamide-based resin as a material. Therefore, as shown in FIG. 5, upon thermal fusion, the interface which bonds the inner layer 324a and the outer layer 324b with each other is peeled off, the peel-off causes the outer layer 324b in the proximal end side of the inner tube 324 to be in a free state with respect to the inner layer 324a, and the outer layer 324b becomes a burr due to, for example, curling or projecting in a radial direction in the proximal end side. Then, depending on the case, the outer layer 324b may be detached.

However, in the invention, in the proximal end side of the inner tube 324, the connection tube 325 is disposed, and the fused part 326 is formed by thermal fusion between an end 324d in the proximal end side of the inner tube 324 (particularly, the end 324d of the outer layer 324b) and the connection tube 325. Therefore, even if the inner layer 324a and the outer layer 324b are peeled off in the proximal end side of the inner tube 324, the peeled-off outer layer 324b can be prevented from becoming a free state. By virtue of this, generation of burrs in the proximal end side of the inner tube 324 can be prevented.

The hardness of the connection tube 325 is lower than that of the first outer tube 322 and the second outer tube 323 as described above and is lower than the hardness of the outer layer 324b of the inner tube 324. Therefore, the connection tube 325 follows bending, etc. of the guide wire 11 better. By virtue of that, the manipulability of the balloon catheter 10 can be further improved.

### <Modification Example>

Hereinabove, the balloon catheter 10 according to an embodiment of the present invention has been described. However, the present invention can be variously modified other than that. Hereinafter, that will be described.

In the above described embodiment, as a tube member, the first outer tube 322 and the second outer tube 323, which are separately independent, are used and are thermally fused to integrate them. However, as the tube member, a single outer tube in a state in which the guide part 322c formed by a slope is formed in advance may be used. Alternatively, as the tube member, three or more outer tubes may be used for formation by joining them by, for example, thermal fusion.

### [Reference Signs List]

10 ··· BALLOON CATHETER
11 ··· GUIDE WIRE
20 ··· HUB
21 ··· COMMUNICATION PIPE LINE
30 ··· SHAFT MAIN-BODY PART
31 ··· HYPOTUBE PART
32 ··· DISTAL TUBE PART
33 ··· CORE WIRE
40 ··· BALLOON
50 ··· THERMAL FUSION DEVICE
51 ··· UPPER MOLD
52 ··· LOWER MOLD
311, 321 ··· TUBE PIPE LINES
322 ··· FIRST OUTER TUBE (CORRESPONDING TO PART OF TUBE MEMBER)
322b, 323b, 324c, 325a ··· LUMEN PARTS
323 ··· SECOND OUTER TUBE (CORRESPONDING TO PART OF TUBE MEMBER)
324 ··· INNER TUBE
324a ··· INNER LAYER
324b ··· OUTER LAYER
324d ··· END
325 ··· CONNECTION TUBE
326 ··· FUSED PART
330 ··· GUIDE WIRE PORT

## Claims

1. A balloon catheter (10) comprising:
an expandable balloon (40);
a hypotube (31) extended in a longitudinal direction;
a tube member (322, 323) extended in the longitudinal direction, connected to a proximal end side of the balloon (40) and a distal end side of the hypotube (31), and having, at a side surface, a guide wire port (330) for inserting a guide wire (11);
an inner tube (324) having at least part disposed between the balloon (40) and the guide wire port (330) in the tube member (322, 323) and having a frictional coefficient of an inner surface lower than that of the tube member (322, 323); and
a connection tube (325) communicated with the inner tube (324), having a proximal end side disposed in the vicinity of the guide wire port (330), and having hardness lower than hardness of the tube member (322, 323), **characterized in that**
a connection tube (325) is disposed in the proximal end side of the inner tube (324), and
a fused part (326) is formed by thermal fusion between an end (324d) in the proximal end side of the inner tube (324) and the connection tube (325).

2. The balloon catheter (10) of claim 1, wherein
the tube member (322, 323) and the connection tube (325) are formed by using a polyamide-based resin as a material;
an inner layer (324a) of the inner tube (324) is formed by using a polyethylene-based resin or a fluorine-based resin as a material, and an outer layer (324b) of the inner tube (324) is formed by using a polyamide-based resin as a material; and,
between the tube member (322, 323), the outer layer (324b) of the inner tube (324), and the connection tube (325), the fused part (326) at which these are fused is present.

3. The balloon catheter (10) of claim 1 or 2, wherein the hardness of the connection tube (325) is lower than the hardness of the outer layer (324b) of the inner tube (324).

## Patentansprüche

1. Ballonkatheter (10) mit:
einem expandierbaren Ballon (40);
einer Hypo-Röhre (31), die sich in einer Längsrichtung erstreckt;
einem Röhrenelement (322, 323), das sich in der Längsrichtung erstreckt, mit einer proximalen Endseite des Ballons (40) und einer distalen Endseite der Hypo-Röhre (31) verbunden ist und an einer Seitenfläche einen Führungsdrahtanschluss (330) zum Einsetzen eines Führungsdrahtes (11) hat;
einer inneren Röhre (324), die zumindest einen Teil hat, der zwischen dem Ballon (40) und dem Führungsdrahtanschluss (330) in dem Röhrenelement (322, 323) angeordnet ist, und die einen Reibungskoeffizienten einer Innenfläche hat, der kleiner ist als jener des Röhrenelements (322, 323); und
einer Verbindungsröhre (325), die mit der inneren Röhre (324) in Verbindung ist, eine proximale Endseite hat, die in der Nähe des Führungsdrahtanschlusses (330) angeordnet ist, und eine Härte hat, die kleiner ist als eine Härte des Röhrenelements (322, 323),
**dadurch gekennzeichnet, dass**
eine Verbindungsröhre (325) an der proximalen Endseite der inneren Röhre (324) angeordnet ist, und
ein verschweißter Teil (326) durch Wärmeschweißen zwischen einem Ende (324d) an der proximalen Endseite der inneren Röhre (324) und der Verbindungsröhre (325) ausgebildet ist.

2. Ballonkatheter (10) gemäß Anspruch 1, wobei
das Röhrenelement (322, 323) und die Verbindungsröhre (325) unter Verwendung eines Kunststoffes auf Polyamidbasis als Material ausgebildet ist;
eine innere Schicht (324a) der inneren Röhre (324) unter Verwendung eines Kunststoffes auf Polyethylenbasis oder eines Kunststoffes auf Fluorbasis als Material ausgebildet ist, und eine äußere Schicht (324b) der inneren Röhre (324) unter Verwendung eines Kunststoffes auf Polyamidbasis als Material ausgebildet ist; und
zwischen dem Röhrenelement (322, 323), der äußeren Schicht (324b) der inneren Röhre (324) und der Verbindungsröhre (325) der verschweißte Teil (326) vorhanden ist, an dem diese verschweißt sind.

3. Ballonkatheter (10) gemäß Anspruch 1 oder 2, wobei die Härte der Verbindungsröhre (325) kleiner ist als die Härte der äußeren Schicht (324b) der inneren Röhre (324).

## Revendications

1. Cathéter à ballonnet (10) comprenant :
un ballonnet expansible (40) ;
un hypotube (31) étendu dans une direction longitudinale ;
un élément tubulaire (322, 323) étendu dans la direction longitudinale, raccordé à un côté d'extrémité proximale du ballonnet (40) et un côté d'extrémité distale de l'hypotube (31), et ayant, sur une surface latérale, un orifice pour fil guide (330) pour insérer un fil guide (11) ;
un tube interne (324) ayant au moins une partie disposée entre le ballonnet (40) et l'orifice pour fil guide (330) dans l'élément tubulaire (322, 323) et ayant un coefficient de friction d'une surface interne inférieure à celle de l'élément tubulaire (322, 323) ; et
un tube de raccordement (325) en communication avec le tube interne (324), ayant un côté d'extrémité proximale disposé à proximité de l'orifice pour fil guide (330) et ayant une dureté inférieure à la dureté de l'élément tubulaire (322, 323),
**caractérisé en ce que**
un tube de raccordement (325) est disposé dans le côté d'extrémité proximale du tube interne (324), et
une partie fusionnée (326) est formée par fusion thermique entre une extrémité (324d) dans le côté d'extrémité proximale du tube interne (324) et le tube de raccordement (325).

2. Cathéter à ballonnet (10) selon la revendication 1, dans lequel
l'élément tubulaire (322, 323) et le tube de raccordement (325) sont formés en employant une résine à base de polyamide comme matériau ;
une couche interne (324a) du tube interne (324) est formée en employant une résine à base de polyéthylène ou une résine à base de fluor comme matériau, et une couche externe (324b) du tube interne (324) est formée en employant une résine à base de polyamide comme matériau ; et
entre l'élément tubulaire (322, 323), la couche externe (324b) du tube interne (324) et le tube de raccordement (325), la partie fusionnée (326) à laquelle ceux-ci sont fusionnés est présente.

3. Cathéter à ballonnet (10) selon la revendication 1 ou 2, dans lequel la dureté du tube de raccordement (325) est inférieure à la dureté de la couche externe (324b) du tube interne (324).
